Europäisches Patentamt

European Patent Office

Office européen des brevets

(19)

(11) Publication number: **0 148 607**
**A1**

## (12) EUROPEAN PATENT APPLICATION

(21) Application number: **84308685.1**

(22) Date of filing: **13.12.84**

(51) Int. Cl.⁴: **B 01 D 53/14**

(30) Priority: **19.12.83 US 562654**

(43) Date of publication of application: **17.07.85**
**Bulletin 85/29**

(84) Designated Contracting States: **BE DE FR GB IT NL**

(71) Applicant: **THE DOW CHEMICAL COMPANY, Dow Center 2030 Abbott Road Post Office Box 1967, Midland Michigan 48640 (US)**

(72) Inventor: **Pearce, Roscoe L., 313 Narcissus, Lake Jackson, Texas 77566 (US)**
Inventor: **Wolcott, Richard A., 103 Finch, Angleton, Texas 77515 (US)**

(74) Representative: **Raynor, John et al, W.H.Beck, Greener & Co 7 Stone Buildings Lincoln's Inn, London WC2A 3SZ (GB)**

(54) **Use of nucleating agents for enhancement of efficiency of acid gas scrubbing processes.**

(57) A process and plant for enhancing the efficiency of acid gas scrubbing processes in which sorbent is regenerated. The sorbent is contacted with a nucleating agent, for example carbon particles, and optionally a stripping gas, for example natural gas, steam, nitrogen, or $CO_2$ prior to its regeneration.

EP 0 148 607 A1

USE OF NUCLEATING AGENTS FOR

ENHANCEMENT OF EFFICIENCY

OF ACID GAS SCRUBBING PROCESSES


The removal of acid gases such as $H_2S$, $SO_2$, $CO_2$ and the like from natural and synthetic gases containing the same using a sorbent for the acid gas(es), regenerating the sorbent to release the acid gas(es) and returning a lean sorbent to the acid gas removal step is an old and well documented technology. The nature of the sorbents is wide and includes hot potassium carbonate, the alkanolamines, the glycols, as well as sulfinol, and the like to name but a few. Each of these sorbents has a particular advantage for specific acid gases or natural or synthetic gas composition, physical condition of the gas containing the same, as well as purpose of the removal, i.e., clean up a gas stream for combustion or chemical usage or recovery of a gas from the other components, for example $CO_2$ from a flue gas.

Recent pressures on treatment or recovery costs in light of escalating gas prices, environmental concerns, and/or potential large volume uses of a component, e.g., $CO_2$, if price is low, have prompted modification of the commercially practiced process to increase the loadings of the sorbents and/or increase the acid gas removal capacity or gas recovery capacity in existing plants by using higher concentrations of sorbents as well as more difficulty regenerated sorbents. These modified processes usually require higher heat duty in the regeneration units, in many cases resulting in, in addition to higher fuel costs, more severe corrosion problems.

It is therefore an object of the present invention to provide a means to more effectively utilize these modified processes while reducing costs and corrosion problems attendant the regenerator sections in the modified versions.

In accordance with the present invention, a rich sorbent solution, for example potassium carbonate, an alkanolamine such as monoethanolamine, a glycol, or sulfinol, can have the acid gas content reduced prior to regeneration by contacting the rich sorbent with a nucleating agent (eg. carbon particles), with or without subjecting the rich sorbent to gas stripping. The effect of these two simple operations flashes a considerable amount of the acid gases held by the sorbent. These acid gases can be combusted if the stripping gas is a combustible gas, or recovered or reacted as in a Claus tail gas unit. The result of flashing part of the acid gas content of the rich sorbent is that less heat is required to regenerate the sorbent, in certain instances, reduces the load on tail gas recovery or conversion units, and reduces the corrosion potential in the regeneration section.

32,576-F

Substantially any nucleating agent may be employed so long as it does not react with the sorbent or the acid gas(es) being nucleated. One can employ carbon particles, activated carbon or charcoal particles, ceramic particles, wire meshes, non--woven mat or packings and the like.

Similarly, the nature of the stripping gas is not critical so long as it does not react with either the sorbent or the acid gas(es). One can thus employ natural gas, treated or untreated, synthetic gas(es), steam, nitrogen, carbon dioxide if $CO_2$ is not the gas being sorbed, and the like.

It is also to be understood that although the nucleating agent may be employed alone, improved results are obtained when the stripping gas is also employed.

It is further to be understood that the operations may be carried out individually in different vessels, if so, preferably with the nucleating step being the first.

Several modifications in existing commercial gas treating plants are illustrated in the drawings of which;

Figure 1 represents a flow diagram of a commercial acid gas treating plant incorporating the modification of the present invention;

32,576-F

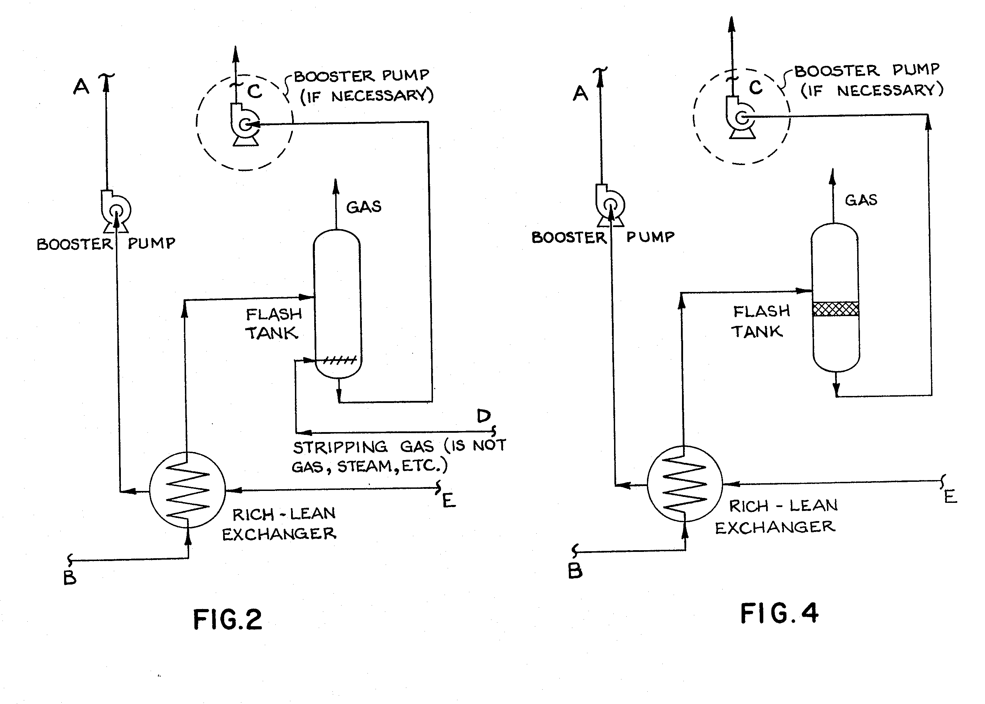

Figure 2 represents the same plant with only the stripping gas being incorporated;

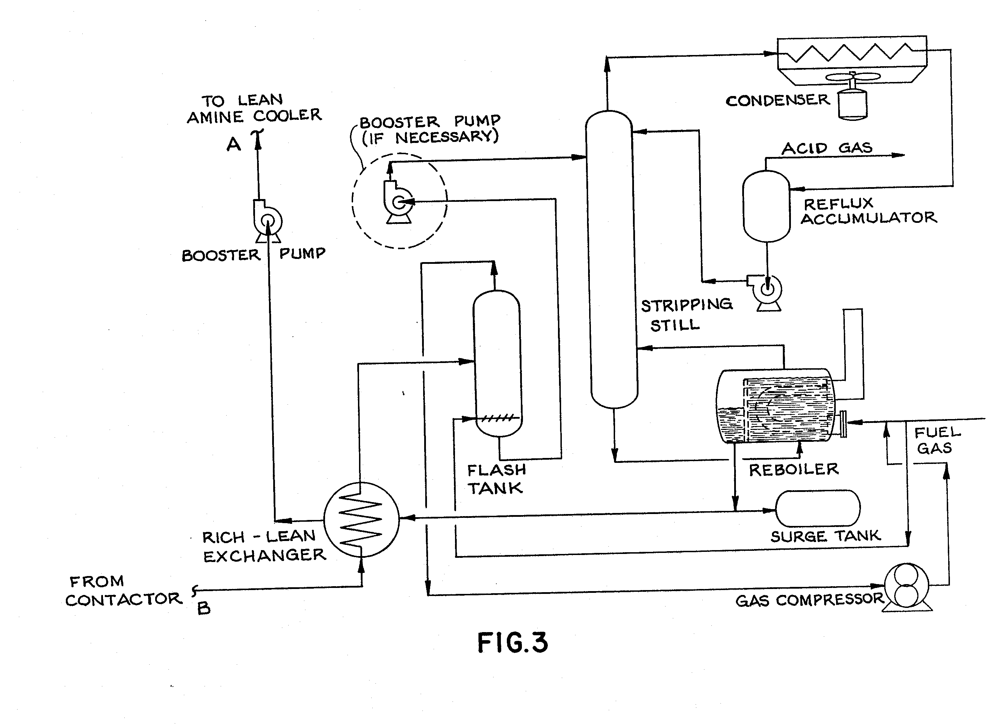

Figure 3 illustrates a flow diagram of the same plant using both nucleation and stripping gas wherein the stripping gas is the fuel gas to the reboiler of the stripping still (regenerator);

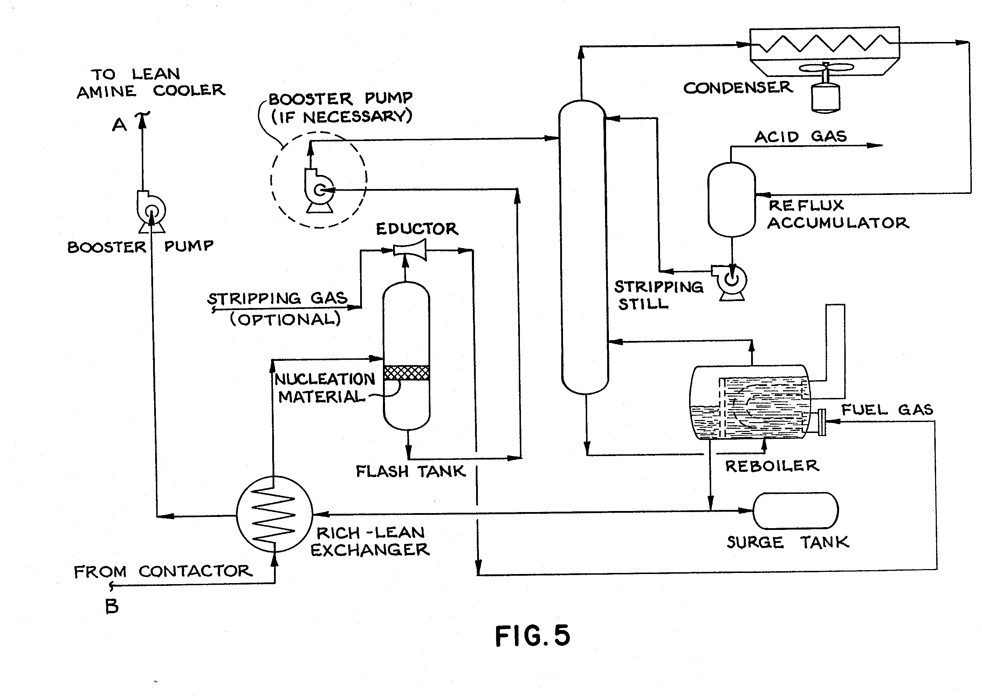

Figure 4 illustrates a commercial plant utilizing only nucleation in accordance with the present invention; and,

Figure 5 illustrates the use of fuel gas to an eductor to decrease the partial pressure over a nucleating bed.

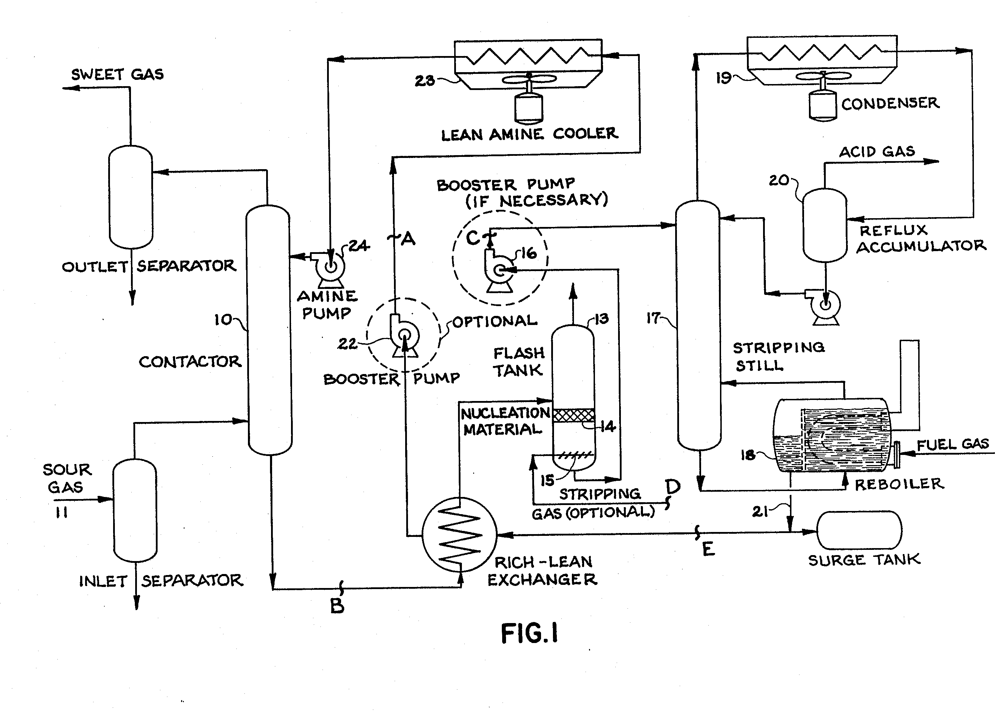

In accordance with the present invention, a gas treating plant as illustrated in Figure 1 consisting of a contactor or absorber (10) which provides a countercurrent contact of a sorbent with the sour gas (11) to be treated, a rich-lean heat exchanger (12) and in accordance with the present invention a rich sorbent flash tank (13) containing the nucleating agent (14) and provisions for distributing the stripping gas (15), optionally a booster pump (16), and a stripping still or regenerator (17) having, (a) a reboiler (18) associated with it to heat the sorbent, and, (b) a condenser (19) to condense the overhead, for return as a liquid the condensed sorbent through (c) an accumulator (20) to the top of the still (17). The hot lean sorbent (21) from the reboiler (18) after passing through the rich-lean

0148607

exchanger (12), a booster pump (22) if necessary, and a lean cooler (23) to cool the sorbent to the best sorbing temperature is returned via pump (24), to the contactor (10).

The modification is simple and can be incorporated with little capital investment. The savings in fuel, corrosion and the like as well as the ability to increase both the concentration of the sorbent solution and the loadings of acid gas in the sorbent, all constitute economic advantages as well as permitting retrofit usage of existing plants many designed for 10-20 percent aqueous sorbent solutions.

The remainder of the modifications are self-evident from the above description and drawings.

CLAIMS

1. A process for removing acid gases from natural or synthetic gases containing the same which process comprises contacting the acid gas-containing stream with a regenerable liquid sorbent solution to produce a rich sorbent solution, and regenerating the sorbent in a manner to release the acid gases sorbed in said rich sorbent solution to produce a lean sorbent, characterised in that the rich sorbent is subjected, prior to its regeneration, to contact with a nucleating agent, to reduce the acid gas content of the rich sorbent solution.

2. A process as claimed in Claim 1, wherein the lean sorbent is recycled to the contacting step.

3. A method as claimed in Claim 1 or Claim 2, further characterised in that the rich sorbent solution is also subjected to contact with a gas, which is non-reactive with the sorbent and the acid gas(es), in a zone wherein acid gases can be flashed from said rich sorbent solution.

4. A process as claimed in Claim 3, wherein the non-reactive gas is natural gas, steam, nitrogen, or $CO_2$.

5. A process as claimed in any one of the preceding claims, wherein the sorbent is an alkanolamine.

6. A method as claimed in Claim 5, wherein the alkanolamine is monoethanolamine.

0148607

7.   A method as claimed in any one of the preceding claims, wherein the nucleating agent comprises carbon particles, ceramic particles, wire mesh, or non-woven matting.

8.   An acid gas treatment plant having (1) a contactor section for treating a gas stream with a sorbent solution to produce a rich sorbent stream, and (2) a regeneration section for removing acid gases from the rich sorbent stream, characterised in that there is interposed between said contactor section and said regeneration section, a nucleation section for contactng the rich sorbent stream with a nucleation agent.

9.   Plant as claimed in Claim 8, further characterised in that there is a stripping gas section, which may be physically integral with said nucleation section, for contacting the rich sorbent solution with a gas which is non-reactive with the sorbent and the acid gas(es).

FIG.1

BOOSTER PUMP (IF NECESSARY)

GAS

FLASH TANK

C

A

BOOSTER PUMP

RICH - LEAN EXCHANGER

B

E

**FIG. 4**

BOOSTER PUMP (IF NECESSARY)

GAS

FLASH TANK

C

A

BOOSTER PUMP

RICH - LEAN EXCHANGER

STRIPPING GAS (IS NOT GAS, STEAM, ETC.)

D

E

B

**FIG. 2**

FIG.3

FIG. 5

0148607

European Patent
Office

**EUROPEAN SEARCH REPORT**

0148607
. Application number

EP 84 30 8685

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-3 222 212 (LINDE AG)<br><br>* Figure 1; page 6, line 5 - page 7, line 30 *<br>--- | 1,2,5-8 | B 01 D 53/14 |
| A | EP-A-0 078 412 (LINDE AG)<br><br>* Figure 1; page 6, line 8 - page 7, line 24 *<br>--- | 1-3,8,9 | |
| A | US-A-2 926 753 (A.L. KOHL)<br><br>----- | | |

TECHNICAL FIELDS
SEARCHED (Int. Cl.4)

B 01 D 53/00

The present search report has been drawn up for all claims

| Place of search | Date of completion of the search | Examiner |
|---|---|---|
| THE HAGUE | 27-03-1985 | BOGAERTS M.L.M. |